# EUROPEAN PATENT SPECIFICATION

(11) **EP 3 684 313 B1**
(45) Date of publication and mention of the grant of the patent: **12.01.2022**
(21) Application number: 18785455.9
(22) Date of filing: 17.09.2018
(51) Int. Cl.: A61F 13/02, A61M 35/00, A61L 15/40, A61F 13/00, A61L 26/00

(54) **WOUND DRESSING DEVICE, ASSEMBLY AND METHOD**
WUNDVERBAND, ANORDNUNG UND VERFAHREN
DISPOSITIF, ENSEMBLE ET MÉTHODE DE PANSEMENT

(30) Priority: 24.09.2017 IL 25464417
(43) Date of publication of application: 29.07.2020
(73) Proprietor: Reddress Ltd., 3701130 Pardes Hana (IL)
(72) Inventor: KUSHNIR, Alon, 3780800 Givat Ada (IL); KUSHNIR, Igal, 3701142 Pardes Hana (IL)
(74) Representative: Grünecker Patent- und Rechtsanwälte PartG mbB
(86) International application number: PCT/IL2018/051049
(87) International publication number: WO 2019/058373

(56) References cited:
- US-A- 6 096 943
- US-A1- 2007 055 205
- US-A1- 2012 179 121
- US-A1- 2015 044 272
- Anonymous: "Telfa Dressings Datacard", , 28 March 2002 (2002-03-28), XP055533497, Retrieved from the Internet: URL:http://www.dressings.org/Dressings/tel fa.html [retrieved on 2018-12-12]
- R. W. Farndale ET AL: "The role of collagen in thrombosis and hemostasis", Journal of Trhombosis and Haemostasis, 1 January 2004 (2004-01-01), pages 561-573, XP055533667, Retrieved from the Internet: URL:https://onlinelibrary.wiley.com/doi/pd f/10.1111/j.1538-7836.2004.00665.x [retrieved on 2018-12-12]
- Monica . Sanandam ET AL: "CHITOSAN BANDAGE FOR FASTER BLOOD CLOTTING AND WOUND HEALING", International Journal of Advanced Biotechnology and Research, 1 January 2013 (2013-01-01), pages 47-50, XP055533663, Retrieved from the Internet: URL:https://www.researchgate.net/profile/K iran_Shejale/publication/263244625_CHITOSA N_BANDAGE_FOR_FASTER_BLOOD_CLOTTING_AND_WO UND_HEALING/links/0046353a41cedb3fdf000000 /CHITOSAN-BANDAGE-FOR-FASTER-BLOOD-CLOTTIN G-AND-WOUND-HEALING.pdf?origin=publication _detail [retrieved on 2018-12-12]

## Description

### TECHNOLOGICAL FIELD

This disclosure is in the field of wound treatment and concerns a wound dressing assembly.

### BACKGROUND

Chronic wounds and skin ulcers are a serious medical condition and effective wound treatment approaches is a recognized medical need.

US 9,180,142 discloses a wound treatment procedure by which blood is coagulated and the so-formed blood clot is applied onto a wound with a dressing material.

### GENERAL DESCRIPTION

The invention is defined as in claim 1. The presence of the features of claim 1 is mandatory, irrespective of the features described as optional in the following.

Reference(s) to "embodiment(s)" throughout the description which are not under the scope of the appended claims merely represent possible exemplary executions and are not part of the present invention.

Similarly, reference(s) to a method of dressing a wound throughout the description are not part of the present invention.

The present disclosure concerns wound treatment through the use of a blood clot. Specifically provided by this disclosure is a wound dressing device and assembly (e.g. in the form of a kit-of-parts for use in the currently disclosed wound treatment) for forming a blood clot, a method for preparing a blood clot-comprising wound dressing, and a method for dressing the wound therewith.

The blood clot that is formed and used according to this disclosure is typically formed from blood of the same subject whose wound is to be dressed by the teaching of this disclosure, withdrawn from the subject in any manner acceptable in medical practice for blood withdrawal. However, the use of blood from a different source, e.g. blood obtained from a blood bank, is also contemplated in accordance with this disclosure.

Three aspects are provided by this disclosure: one concerns a wound dressing device; the other concerns an assembly, e.g. in the form of a kit-of-parts, which comprises said device as one of its components; and the third concerns a method of wound dressing and a use wherein said device is a key component.

All three aspects center around the formation of a blood clot, *in situ,* onto a wound, as part of the wound dressing procedure. In other words, the blood, still in liquid state, is brought into contact with the wound and is induced to clot while at least partially in contact with the wound to be dressed therewith. All three aspects are encompassed in the following description by the term *"wound dressing procedure".*

The method comprises fixing a device of the kind disclosed herein, on top of the wound, the device defining with the wound an enclosed space which serves as a mold (cast) for clotting blood on top of the wound. This space will be referred to herein as a *"mold space".*

The device comprises a cavity or depression surrounded by lips configured for attachment to skin in a fluid tight manner. The cavity or depression may have walls that may be generally concave, polygonal or any other suitable shape. In some embodiments, the cavity is shallow, namely, it has a depth relatively smaller than the overall area confined by the walls.

The lips typically define narrow flat surfaces that when brought into contact with the skin, should be relatively level and smooth to permit fluid tight attachment to the skin. By an embodiment of this disclosure, the lips may carry an adhesive that will ensure fluid tight attachment to the skin.

In addition, in some embodiments, the adhesive may be applied onto the skin or onto the lips, prior to attachment. At times, according to this embodiment, at least a portion of the lips may have a rough surface onto which the adhesive is applied prior to fixation onto the skin. It is also possible, by other embodiments of this disclosure, to fit a two-sided adhesive strip onto the lips or the skin to be used as means for attachment. By still another embodiment, the attachment may be by means of an adhesive tape fitted over the device's lips and the surrounding skin portions for tight association throughout the blood clot formation process.

By other embodiments, the attachment of the lips to the skin may be by forming a vacuum within the mold space, by forcing the device (and hence the lips) against the skin by hand, by the use of an elastic band, adhesive tape or other means for tight forcing of the device against the skin.

The wound's surface area confined between the lips may have different shapes and sizes to suit wounds of different shapes and sizes. Thus, the wound dressing procedure may also involve selection of a device of the proper shape and size to permit the lips to be placed on skin portions surrounding the wound's boundaries. In some embodiments, the device is flexible or pliable to permit its shaping and/or stretching to a desired shape and wound surface area to be confined between the lips.

The device may also comprises a closure that is removably attached to the lips and seals the cavity until use. The closure may be useful for maintaining sterility of the cavity and for holding elements, such as a coagulant initiator and/or scaffold matrix within the cavity. The closure may, for example, be a film made of plastic, foil, a combination thereof, etc. that is pealed from the opening of the cavity (the lips) prior to fixation onto the skin.

according to the wound dressing procedure disclosed herein, once the lips are firmly fixed to skin portions surrounding the wound, a mold space is formed, which, as noted above, is defined between the surface of the wound and the cavity's walls. Blood, typically whole blood, can then be introduced into the mold space and permitted to clot within the mold space to form a blood clot over the wound. The blood clot is maintained over the wound for a time period. This time period may vary and is typically several hours, several days or several weeks, e.g. 1 day, 2-6 days, 1 week, 2-4 weeks, and at times even longer, as well as for any period of time in-between than that indicated. In some embodiments, the blood clot may be maintained over the wound for the entire healing process of several weeks to several month. In some embodiments the wound treatment comprises periodical refreshing procedures that comprises removal of an existing blood clot from the wound and then forming a new blood clot on the wound in a manner as described above. The time period between a blood clot formation over a wound and the performance of a refreshing procedure may be, for example, 1 day, 2-6 days, 1 week, 2-4 weeks, etc., as well as for any period of time in-between than that indicated. The entire wound healing procedure may involve several consecutive refreshing procedures.

The device's walls may be made from a variety of materials. Typically, in order to permit monitoring the blood introduction and the clot formation, the walls or parts thereof are transparent.

Introduction of the blood involves, by one embodiment, piercing the wall with a needle and then injecting blood into the mold space through the needle. Optionally, this introduction is preceded by a second piercing of the wall on an adjacent or different location, to thereby form a vent aperture to permit release of excess pressure during blood introduction. By other embodiments, the walls may comprise a venting valve which, *a priori,* is sealed and opened before the blood introduction and/or a port for introducing the blood into the mold space.

By one embodiment, the device is removed after the blood clot is formed. To facilitate easy removal, e.g. without severing the integrity of the formed blood clot, the walls are preferably made of or coated with a material to which a blood clot does not adhere. By another embodiment, the device or part thereof remains *in situ* after the blood clot is formed and serves as part of the wound dressing.

In the case where the device is removed after clot formation, a dressing material, for example, gauze, plaster or bandage, may be fitted over the blood clot to protect and secure the blood clot in place. The wound dressing procedure, by some embodiments, may make use of a scaffold matrix combined with and brought into contact with the blood prior to its clotting, so as to become integrated with the thus formed blood clot. The scaffold matrix supports the clot and assists in maintaining the clot's structural integrity. The scaffold matrix may, by one embodiment, be an independent element fitted onto the wound, prior to said fixing of the device onto the skin; by another embodiment, the scaffold matrix may be comprised within the cavity, e.g. *a priori* comprised therein or introduced into the cavity before fixing the device onto the wound. The scaffold matrix may, for example, be a 2-domesional or 3-dimensional matrix, may be a porous material, may have a netlike structure; may be made of a polymeric material such as plastic, may be made out of natural or synthetic fibers or made out of a woven or non-woven cloth, e.g. gauze.

In order to ensure controlled coagulation, the blood may be contacted with one or combination of coagulation initiators, namely, one or more substances (synthetic or naturally occurring) that promotes/activates blood coagulation. In some embodiments, the coagulation initiator is kaolin. When using a coagulation initiator, coagulation of blood may involve, for example, mixing the blood with kaolin shortly before introduction into the mold space. Alternatively, the coagulation initiator may *a priori* be present within the cavity; for example, attached to the walls or incorporated into a scaffold matrix, placed within or a priori comprised within the cavity or further, separately introduced into the mold space (e.g. by injection) before or after introduction of the blood. In addition or alternatively, the coagulation initiator may be incorporated into a scaffold matrix that is fitted onto the wound, prior to fixing of the device over the found.

In some embodiments, the coagulation initiator is independently stored, e.g. as a powder, granulate or liquid in a separate container and subsequently mixed with the blood upon introduction into the mold space or applied onto the scaffold matrix prior to or after introducing the blood. By still another alternative, the coagulation initiator may be applied directly onto the wound as a first step in the wound treatment procedure.

The wound dressing assembly or kit of this disclosure comprises a clotting mold device of the kind specified above. In addition, according to some embodiments, the assembly or kit may comprise other elements or devices required for the *in situ* formation of the blood clot on the wound, e.g. a syringe for introducing blood into the cavity. The assembly or kit may comprise also a dressing material for applying over the blood clot formed over the wound.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig 1** shows a wound dressing device according to an embodiment of this disclosure.
**Fig. 2** shows the wound dressing device after removal of the closure and applied onto the skin.
**Figs. 3A and 3B** show, respectively, two steps of introducing blood into the mold space.
**Fig. 4** shows the wound dressing device with blood still in liquid form with the device secured by adhesive tape to the skin.
**Fig. 5** shows the blood clot with the device partially ruptured prior to removal.
**Fig. 6** shows the blood clot on the skin after its formation and after the device has been removed.

### DETAILED DESCRIPTION OF EMBODIMENTS

In the following, reference is made to embodiments shown in the annexed Figures. It should be noted that these Figures are pictures from a test trial on skin without a wound, to demonstrate the wound dressing procedure of this disclosure.

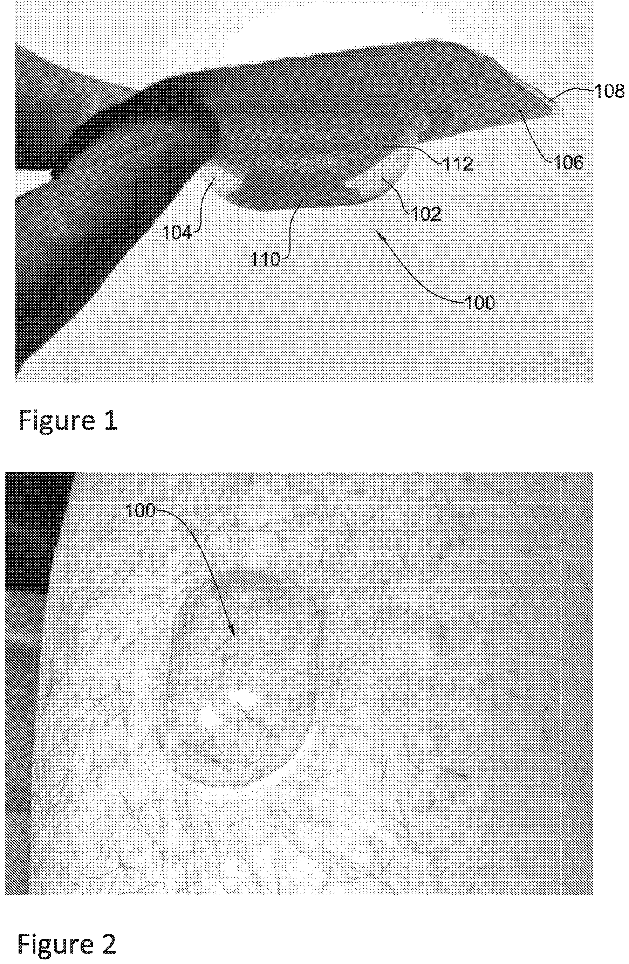

The device **100** shown in **Fig. 1** includes generally concave walls **102** that form a cavity **104** defined between lips **106** that are generally flat. The opening defined between the lips is sealed by a removable closure **108** which is a laminate/film fitted onto lips **106.** After removal of closure **108,** the device **100** is placed onto skin, an adhesive remaining after removal of the closure causes it to adhere to the skin. A device **100** fixed to a subject's skin is shown in Fig. 2.

Also shown in **Fig.1** is a scaffold matrix **112** placed within the cavity, the scaffold matrix holds a coagulation initiator **110.**

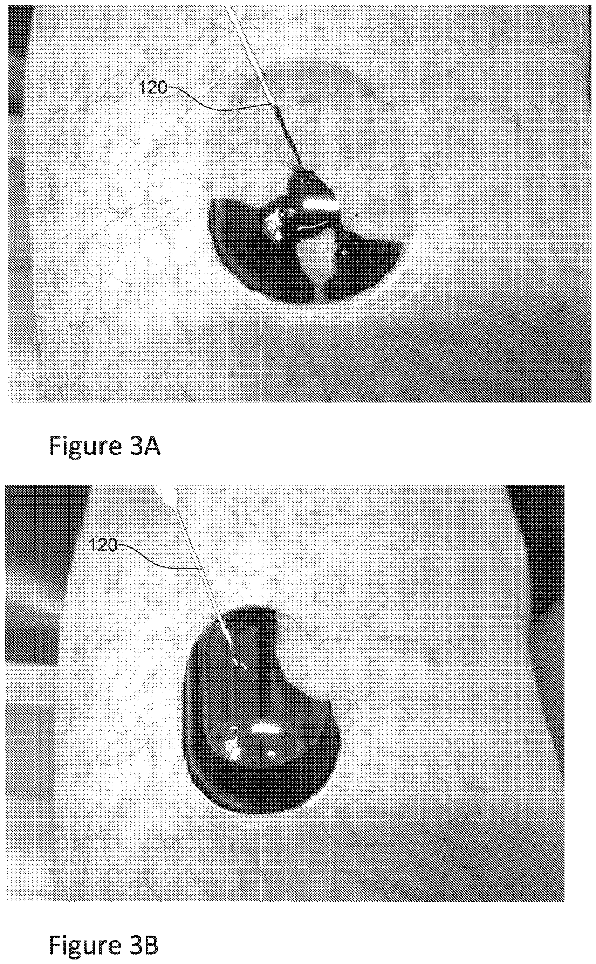

Turning now to **Figs. 3A-3B****,** steps of introducing blood, typically whole blood into the mold space are shown. Specifically, blood withdrawn from the subject or alternatively taken from a blood bank, is injected with a needle **120** into the mold space formed between the walls **102** and underlying portion of the skin or wound. According to this non-limiting embodiment, upon introduction into the mold space, the blood is mixed with the coagulation initiator **110** *a priori* present in the cavity. The needle **120** first pierces the walls of the device and then injects the blood contained in the syringe (not seen).

In some other embodiments, the blood is pre-mixed with a coagulation initiator, such as Kaolin, that is provided in a separate container (not shown), or the coagulation initiator is introduced into the mold space separately, either before, during or after the blood introduction.

As can also be seen in **Figs 1-3B** the walls of the device are transparent to permit monitoring the introduction and subsequent blood clotting.

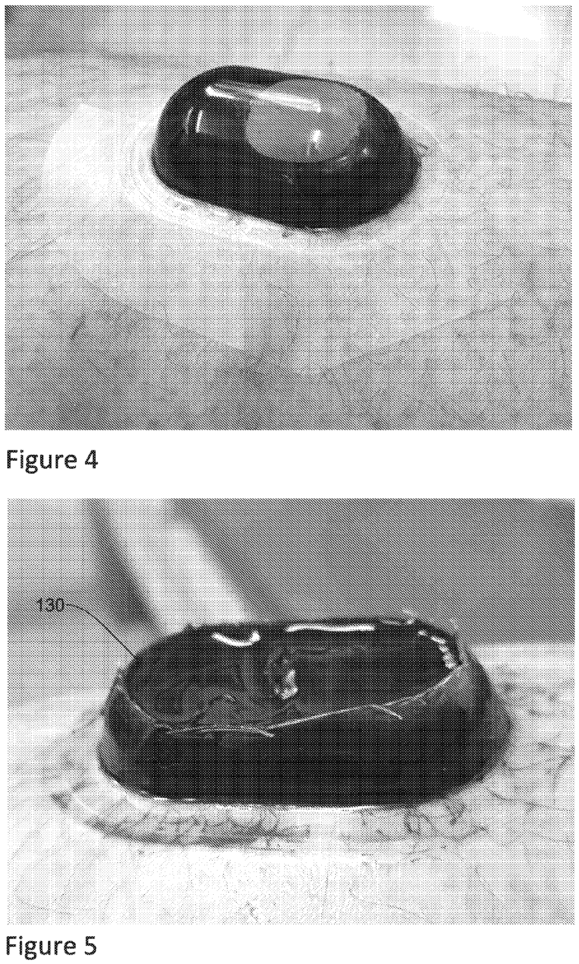

The device is then allowed to remain attached to the skin with the blood held within the mold space for a time at least until the blood has clotted. Such time may range from several days, to one or more weeks, The device can be secured to the skin by use of an adhesive strip **130,** as seen in **Fig. 4****.**

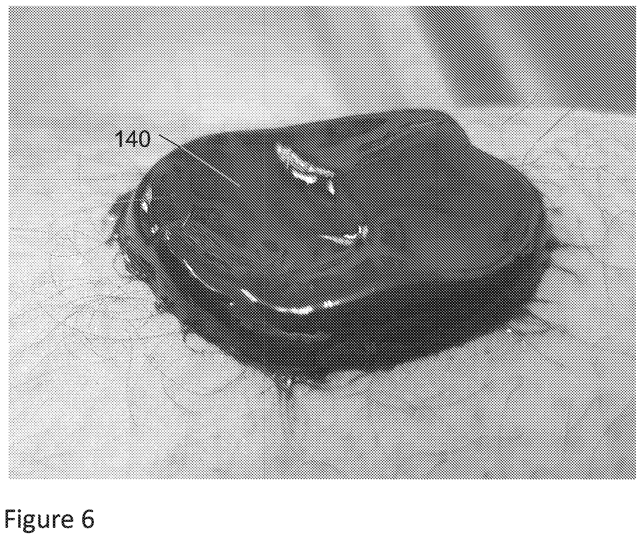

After a period of time, a blood clot is formed and the walls of the device can then be cut away, as seen in **Fig. 5****;** and, after removal, a blood clot **140** remains on top of the skin, as seen in **Fig. 6****.** Where the procedure will be performed over a wound, the blood clot will cover the wound and a dressing material, which may be conventional dressing material, such as gauze or bandage, will be applied over the clot for protecting the wound and securing the clot in place.

## Claims

1. A wound dressing assembly, comprising
a clotting mold device (100) comprising a cavity (104) defined by concave walls (102) surrounded by lips (106) configured for attachment to skin in a fluid tight manner, wherein, for introducing blood into the cavity (104) once said lips (106) are attached to the skin, the concave walls (102) of the clotting mold device (100) comprise one of (i) a portion being pierceable and suitable for introducing blood into the cavity (104), and (ii) a port suitable for introducing said blood into the cavity (104);
a device for introducing blood into the cavity (104) after it is fixed over a wound to permit the blood to come into contact with the wound and to clot over the wound within said cavity (104);
a blood coagulation initiator (110).

2. The wound dressing assembly of claim 1, wherein the device (100) comprises a closure (108) removably fixed to the lips (106) and sealing the cavity (104).

3. The wound dressing assembly of claim 1 or 2, wherein the clotting device (100) comprises a closure (108), e.g. in the form a film, removably fixed to the lips (106) and sealing the mold space.

4. The wound dressing assembly of any one of claims 1 to 3, wherein the lips (106) have an adhesive (130) for adhesion to skin.

5. The wound dressing assembly of any one of claims 1 to 4, comprising a scaffold matrix (112) configured for integration within the formed blood clot (140).

6. The wound dressing of assembly of claim 5, wherein the scaffold matrix (112) is comprised within said cavity (104).

7. The wound dressing assembly of any one of claims 1 to 6, comprising one or both of
a syringe and a needle (120) for piercing the walls (102) and injection of the blood into the cavity (104), and
a dressing material for fixing over the blood clot (140).

8. The wound dressing assembly of any one of claims 1 to 7, wherein said coagulation initiator (110) is in a form of a powder, granulate or solution suitable for mixing with the blood or for applying directly onto the wound, being a priori present in the cavity (104), being incorporated into a scaffold matrix (112) that is comprised within said cavity (104) or intended for placement onto the wound prior to said attachment.

9. The wound dressing assembly of any one of claims 1 to 8, wherein the walls (102) of the cavity (104) comprise one or both of
a vent that is *a priori* sealed, for removal of excess pressure during introduction of the blood into the enclosure, and
a port for introducing blood into the mold space.

10. The wound dressing assembly of any one of claims 1 to 9, wherein the enclosure is made of a material to which a blood clot (140) does not adhere.

11. The wound dressing assembly of any one of claims 1 to 10, wherein at least a portion of the walls (102) is transparent.

## Patentansprüche

1. Wundverbandanordnung, umfassend
eine Koagulationsformvorrichtung (100), umfassend einen Hohlraum (104), der durch konkave Wände (102) definiert ist, die von Lippen (106) umgeben sind, die zur Befestigung an der Haut in einer flüssigkeitsdichten Weise konfiguriert sind, wobei zum Einführen von Blut in den Hohlraum (104), sobald die Lippen (106) an der Haut befestigt sind, die konkaven Wände (102) der Gerinnungsformvorrichtung (100) entweder (i) einen Abschnitt, der durchstechbar und zum Einführen von Blut in den Hohlraum (104) geeignet ist, oder (ii) eine Öffnung, die zum Einführen des Blutes in den Hohlraum (104) geeignet ist, umfassen;
eine Vorrichtung zum Einführen von Blut in den Hohlraum (104), nachdem dieser über einer Wunde befestigt ist, damit das Blut mit der Wunde in Kontakt kommen und über der Wunde innerhalb des Hohlraums (104) koagulieren kann;
einen Blutgerinnungsinitiator (110).

2. Wundverbandanordnung nach Anspruch 1, wobei die Vorrichtung (100) einen Verschluss (108) umfasst, der abnehmbar an den Lippen (106) befestigt ist und den Hohlraum (104) abdichtet.

3. Wundverbandanordnung nach Anspruch 1 oder 2, wobei die Koagulationsvorrichtung (100) einen Verschluss (108), z. B. in Form einer Folie, umfasst, der abnehmbar an den Lippen (106) befestigt ist und den Formraum abdichtet.

4. Wundverbandanordnung nach einem der Ansprüche 1 bis 3, wobei die Lippen (106) einen Klebstoff (130) zur Haftung an der Haut aufweisen.

5. Wundverbandanordnung nach einem der Ansprüche 1 bis 4, umfassend eine Gerüstmatrix (112), die zur Integration in das gebildete Blutgerinnsel (140) konfiguriert ist.

6. Wundverbandanordnung nach Anspruch 5, wobei die Gerüstmatrix (112) in dem Hohlraum (104) enthalten ist.

7. Wundverbandanordnung nach einem der Ansprüche 1 bis 6, umfassend eines oder beides von
eine Spritze und eine Nadel (120) zum Durchstechen der Wände (102) und zum Einspritzen des Blutes in den Hohlraum (104), und
ein Verbandsmaterial zur Fixierung über dem Blutgerinnsel (140).

8. Wundverbandanordnung nach einem der Ansprüche 1 bis 7, wobei der Koagulationsinitiator (110) in Form eines Pulvers, Granulats oder einer Lösung vorliegt, das/die zum Mischen mit dem Blut oder zum direkten Aufbringen auf die Wunde geeignet ist, a priori in dem Hohlraum (104) vorhanden ist, in eine Gerüstmatrix (112) eingearbeitet ist, die in dem Hohlraum (104) enthalten ist, oder dazu bestimmt ist, vor dem Anbringen auf die Wunde gelegt zu werden.

9. Wundverbandanordnung nach einem der Ansprüche 1 bis 8, wobei die Wände (102) des Hohlraums (104) eines oder beide der folgenden Elemente umfassen
eine Entlüftungsöffnung, die a priori abgedichtet ist, um den Überdruck während der Einführung des Blutes in das Gehäuse abzubauen, und
eine Öffnung zum Einführen von Blut in den Formraum.

10. Wundverbandanordnung nach einem der Ansprüche 1 bis 9, wobei die Umhüllung aus einem Material hergestellt ist, an dem ein Blutgerinnsel (140) nicht haftet.

11. Wundverbandanordnung nach einem der Ansprüche 1 bis 10, wobei mindestens ein Teil der Wände (102) transparent ist.

## Revendications

1. Ensemble pansement de plaie, comprenant
un dispositif moule de coagulation (100) comprenant une cavité (104) définie par des parois concaves (102) entourées de lèvres (106) conçues pour la fixation à la peau d'une manière étanche aux liquides, pour l'introduction du sang dans la cavité (104) une fois que lesdites lèvres (106) sont fixées à la peau, les parois concaves (102) du dispositif moule de coagulation (100) comprenant l'un de (i) une portion pouvant être percée et convenant à l'introduction de sang dans la cavité (104), et (ii) d'un orifice convenant à l'introduction dudit sang dans la cavité (104) ;
un dispositif d'introduction de sang dans la cavité (104) après qu'il soit fixé sur une plaie pour permettre au sang d'entrer en contact avec la plaie et de coaguler sur la plaie à l'intérieur de ladite cavité (104) ;
un initiateur de coagulation (110) du sang.

2. Ensemble pansement de plaie selon la revendication 1, le dispositif (100) comprenant une enceinte (108) fixée de manière amovible aux lèvres (106) et fermant la cavité (104) de manière étanche.

3. Ensemble pansement de plaie selon la revendication 1 ou 2, le dispositif de coagulation (100) comprenant une enceinte (108), par exemple sous la forme d'un film, fixée de manière amovible aux lèvres (106) et fermant de manière étanche l'espace moule.

4. Ensemble pansement de plaie selon l'une quelconque des revendications 1 à 3, les lèvres (106) ayant un adhésif (130) pour l'adhésion à la peau.

5. Ensemble pansement de plaie selon l'une quelconque des revendications 1 à 4, comprenant une matrice de charpente (112) conçue pour l'intégration à l'intérieur du caillot de sang (140) formé.

6. Pansement de plaie de l'ensemble selon la revendication 5, la matrice de charpente (112) étant comprise à l'intérieur de ladite cavité (104).

7. Ensemble pansement de plaie selon l'une quelconque des revendications 1 à 6, comprenant l'un ou plusieurs de
une seringue et une aiguille (120) pour percer les parois (102) et l'injection du sang dans la cavité (104), et
un matériau de pansement pour la fixation sur le caillot de sang (140).

8. Ensemble pansement de plaie selon l'une quelconque des revendications 1 à 7, ledit initiateur de coagulation (110) se présentant sous une forme de poudre, de granulat ou de solution convenant au mélange avec le sang ou à l'application directement sur la plaie, étant a *priori* présent dans la cavité (104), étant incorporé dans une matrice de charpente (112) qui est comprise à l'intérieur de ladite cavité (104) ou prévu pour la mise en place sur la plaie avant ladite fixation.

9. Ensemble pansement de plaie selon l'une quelconque des revendications 1 à 8, les parois (102) de la cavité (104) comprenant l'un ou plusieurs de
un orifice de ventilation qui est *a priori* étanche, pour l'élimination de la pression en excès durant l'introduction du sang dans l'enceinte, et
un orifice d'introduction du sang dans l'espace moule.

10. Ensemble pansement de plaie selon l'une quelconque des revendications 1 à 9, l'enceinte étant constituée d'un matériau auquel un caillot de sang (140) n'adhère pas.

11. Ensemble pansement de plaie selon l'une quelconque des revendications 1 à 10, au moins une portion des parois (102) étant transparente.
